# EUROPEAN PATENT APPLICATION

(11) **EP 4 420 785 A1**
(43) Date of publication of application: **28.08.2024**
(21) Application number: 21966583.3
(22) Date of filing: 07.12.2021
(51) Int. Cl.: B01L 7/04, C12M 1/00

(54) **TEMPERATURE CONTROL DEVICE**

(71) Applicant: HITACHI HIGH-TECH CORPORATION, Tokyo 105-6409 (JP)
(72) Inventor: SATO Wataru, Tokyo 100-8280 (JP); SATO Koma, Tokyo 100-8280 (JP); MAKINO Yoko, Tokyo 105-6409 (JP); ISOSHIMA Nobuyuki, Tokyo 105-6409 (JP); SHIBAHARA Masashi, Tokyo 105-6409 (JP)
(74) Representative: Manitz Finsterwald Patent- und Rechtsanwaltspartnerschaft mbB
(86) International application number: PCT/JP2021/044911
(87) International publication number: WO 2023/105627

(57) **Abstract**

An object of the invention is to provide a temperature control device that prevents occurrence of dew condensation on an upper surface of a cover portion. For this purpose, the invention provides a temperature control device including: a thermally conductive portion; a cooling part configured to cool the thermally conductive portion; and a cover portion having an opening through which a container is inserted and removed while covering an upper side of the thermally conductive portion, in which an air layer is formed between the thermally conductive portion and the cover portion. When such a temperature control device is used for a genetic testing device, the dew condensation is unlikely to occur on an upper surface of the cover portion of the temperature control device, and contamination of a sample due to splashing of dew condensation water is prevented. Therefore, it is possible to maintain high testing accuracy.

## Description

### Technical Field

The present invention relates to a temperature control device.

### Background Art

There is a genetic testing device that fully automatically implements extraction of a sample containing Deoxyribonucleic acid (DNA), mixing of reagents, amplification and testing of the DNA. The genetic testing device generally includes a temperature control device that keeps a sample or a reagent at a low temperature.

For example, Patent Literature 1 discloses a sample thermostatic device including a sample rack having a plurality of through holes into which sample bottles are loaded from an upper surface side, and a rack holder including a cooling element such as a Peltier element and a good thermally conductive material in contact with the element.

### Citation List

### Patent Literature

PTL 1: JPH10-192719A

### Summary of Invention

### Technical Problem

In a related-art temperature control device, when cooling a sample or the like, dew condensation may occur due to ambient temperature and humidity, or a temperature of a cooling part. For example, Patent Literature 1 described above proposes a method in which a discharge pipe for discharging accumulated dew condensation water is provided on a bottom surface side of the rack holder. However, in the temperature control device disclosed in Patent Literature 1 described above, since the good thermally conductive material is also brought into contact with a lower surface of the sample rack, the sample rack itself is also likely to be cooled, and the dew condensation occurs on an upper surface of the sample rack. Therefore, in the temperature control device disclosed in Patent Literature 1, a sample may be contaminated, and testing accuracy may decrease due to splashing of the dew condensation water when inserting and removing a sample bottle.

The invention has been made to solve such problems, and an object thereof is to provide a temperature control device that prevents occurrence of dew condensation on an upper surface of a cover portion.

### Solution to Problem

Provided is a temperature control device including: a thermally conductive portion; a cooling part configured to cool the thermally conductive portion; and a cover portion having an opening through which a container is inserted and removed while covering an upper side of the thermally conductive portion, in which an air layer is formed between the thermally conductive portion and the cover portion.

### Advantageous Effects of Invention

According to the invention, it is possible to provide a temperature control device that prevents occurrence of dew condensation on an upper surface of a cover portion. Problems, configurations, and effects other than those described above will be made clear by the following description.

### Brief Description of Drawings

[FIG. 1] FIG. 1 is a schematic perspective view showing a temperature control device according to Embodiment 1.
[FIG. 2] FIG. 2 is a cross-sectional view taken along a line A-A' in FIG. 1.
[FIG. 3] FIG. 3 is a schematic perspective view showing a temperature control device according to Embodiment 2.
[FIG. 4] FIG. 4 is a schematic side view showing the temperature control device according to Embodiment 2.
[FIG. 5] FIG. 5 is a cross-sectional view taken along a line B-B' in FIG. 3.
[FIG. 6] FIG. 6 is an enlarged cross-sectional view showing a shape of a convex portion in FIG. 5 in an enlarged manner.
[FIG. 7] FIG. 7 is a schematic cross-sectional view showing a temperature control device according to Embodiment 3.
[FIG. 8] FIG. 8 is a schematic cross-sectional view showing a temperature control device according to Embodiment 4.

### Description of Embodiments

Hereinafter, embodiments of the invention will be described using Embodiments 1 to 4.

### Embodiment 1

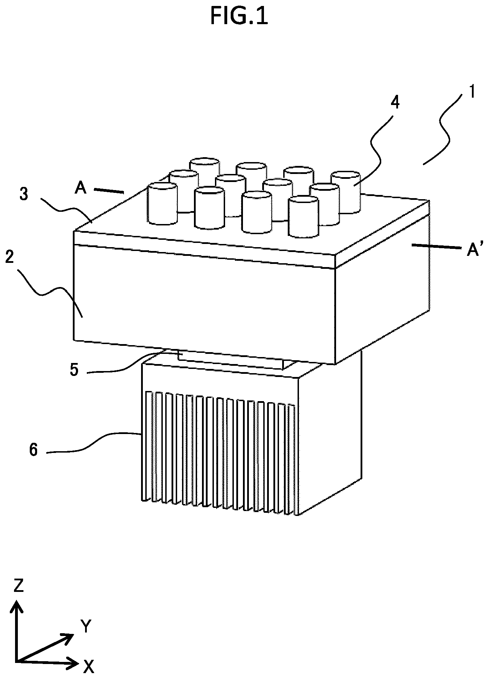

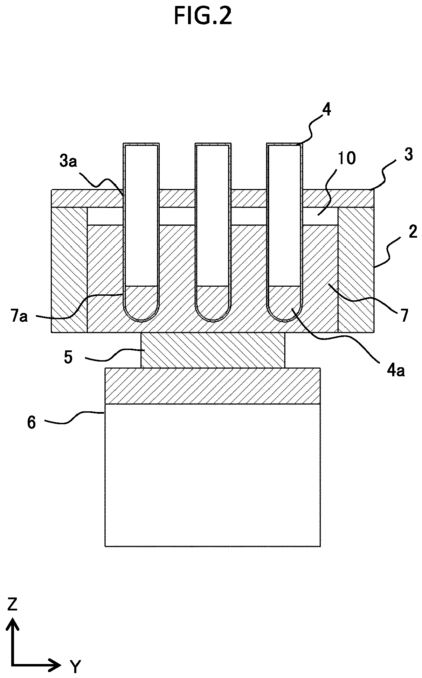

FIG. 1 is a schematic perspective view showing a temperature control device 1 according to Embodiment 1. FIG. 2 is a cross-sectional view taken along a line A-A' in FIG. 1. As shown in FIGS. 1 and 2, the temperature control device 1 according to the present embodiment includes a temperature control block 7 (thermally conductive portion), a temperature control part 5 (cooling part) that cools the temperature control block 7, and a cover portion 3 that covers an upper side of the temperature control block 7.

Here, the temperature control block 7 is formed of aluminum, and has a plurality of concave portions 7a for holding sample containers 4. However, the material of the temperature control block 7 is not limited to aluminum as long as it is a metal having high thermal conductivity, and copper, magnesium alloy, and the like can also be used. It is desirable that a depth of the concave portion 7a formed in the temperature control block 7 is larger than a liquid surface height of a solution 4a in order to satisfactorily maintain cooling performance of the solution 4a contained in the sample container 4. In addition, although not shown, a temperature sensor is disposed in the temperature control block 7.

The temperature control part 5 is a Peltier element in contact with a lower surface of the temperature control block 7. The Peltier element has a function of adjusting a temperature of the solution 4a in the sample container 4 to a predetermined temperature by cooling the temperature control block 7, and specifically, adjusts an output such that a temperature measured by the temperature sensor in the temperature control block 7 becomes the predetermined temperature. However, since the Peltier element generates heat when the temperature control block 7 is cooled, a heat dissipation part 6 that dissipates the heat is required. Although not shown, the heat dissipation part 6 has a structure that dissipates heat to outside air by a fin or a fan. The temperature control part 5 is not limited to the Peltier element, and may introduce cold water or hot water from a heat pump, a chiller, or the like, or may have a structure that combines a plurality of these components.

The cover portion 3 has an opening portion 3a for inserting and removing the sample container 4 such that a horizontal position coincides with that of the concave portion 7a provided in the temperature control block 7. The sample container 4 inserted through the opening portion 3a of the cover portion 3 is supported by the concave portion 7a of the temperature control block 7. It is desirable that the cover portion 3 is formed of a material having thermal conductivity lower than that of the temperature control block 7, for example, resin or rubber. In addition, the cover portion 3 does not need to be formed of only one kind of material, and may be formed of a combination of two or more kinds of materials.

The temperature control device 1 according to the present embodiment includes a heat insulation material 2 that covers a lateral side of the temperature control block 7. The heat insulation material 2 is, for example, a foam heat insulation material, and has a heat insulation performance higher than that of the cover portion 3. Therefore, different from an upper end surface of the temperature control block 7, an upper end surface of the heat insulation material 2 is in contact with the cover portion 3.

Next, an air layer 10 formed between the temperature control block 7 and the cover portion 3 will be described. As shown in FIG. 2, the upper end surface of the temperature control block 7 and a lower surface of the cover portion 3 are separated from each other, and a space is defined by the temperature control block 7, the heat insulation material 2, and the cover portion 3 (and the sample containers 4). The air layer 10 existing in the space serves as a heat insulation layer, and prevents heat of the cover portion 3 from being transmitted to the temperature control block 7. Therefore, in the temperature control device 1, even if the temperature control block 7 is cooled to a temperature lower than that of the outside air, a surface temperature of the cover portion 3 exposed to the outside air is maintained higher than a temperature of the temperature control block 7. That is, even if a temperature of the temperature control block 7 is a temperature lower than a dew point of the outside air, since the surface temperature of the cover portion 3 is maintained at a temperature higher than the dew point of the outside air, dew condensation is unlikely to occur on an upper surface of the cover portion 3.

Here, a desirable thickness of the air layer 10 will be examined. As a premise, it is assumed that a general resin material is used for the cover portion 3, a thickness thereof is 5 mm, and thermal conductivity thereof is 0.2 W/m•K, that is, thermal resistance is 2.5 K/W. Further, as conditions under which the dew condensation is likely to occur, it is assumed that a temperature of the outside air is 30 °C, and humidity of the outside air is 800, that is, a dew point temperature of the outside air is 26.2 °C. Further, it is assumed that thermal conductivity of the air layer 10 is 0.026 W/m•K, a heat transfer coefficient due to natural convection from a surface of the temperature control block 7 to air is simply 15 W/m2•K, and a surface area of the temperature control block 7 is 0.01m2. In addition, it is assumed that a set temperature of the temperature control block 7 is 15 °C.

Under such preconditions, when the thickness of the air layer 10 is 5 mm, a thermal resistance of the air layer 10 is 19.2 K/W, and an equivalent thermal resistance to the air due to the natural convection is 6.67 K/W. When the surface temperature of the cover portion 3 is calculated based on the values of the thermal resistances, it is estimated to be 26.5 °C. That is, since the surface temperature of the cover portion 3 is higher than the dew point temperature of the outside air of 26.2 °C, it is possible to prevent occurrence of the dew condensation on the upper surface of the cover portion 3.

The desirable thickness of the air layer 10 varies depending on the material and the thickness of the cover portion 3, the set temperature of the temperature control block 7, and the like, but an upper limit value may be 10 mm. The reason for this is that when the air layer 10 is thicker than 10 mm, heat transfer due to natural convection in the air layer 10 may increase, and cooling of the cover portion 3 may proceed excessively.

In the examination described above, only the natural convection in the air layer 10 is considered, but actually, it is also necessary to consider an influence of heat transfer due to radiation. Therefore, the lower surface of the cover portion 3 (a surface facing the temperature control block 7) and the upper end surface of the temperature control block 7 (a surface facing the cover portion 3) are formed of materials having low emissivity, whereby it is also possible to prevent an influence of heat transfer due to radiation between the cover portion 3 and the temperature control block 7. For example, it is considered to metal-plate the lower surface of the cover portion 3, or stack thin metal plates or films.

As described above, according to the present embodiment, since the air layer 10 exists between the temperature control block 7 and the cover portion 3, a decrease in the surface temperature of the cover portion 3 is prevented, and it is possible to prevent occurrence of the dew condensation on the upper surface of the cover portion 3. In addition, since outside air containing water vapor other than the air that exists in advance as the air layer 10 is not supplied to the upper end surface of the temperature control block 7, an amount of dew condensation that occurs on the upper end surface of the temperature control block 7 can also be reduced.

The cover portion 3 and the heat insulation material 2 may not be formed separately, and may be formed integrally with common foam or the like. Even if the cover portion 3 is formed of a material having high heat insulation performance such as foam, as compared with a side surface, it is difficult to thicken the upper surface, and existence of the dew condensation is likely to influence inserting and removing the sample container 4. Therefore, the air layer 10 that separates the upper end surface of the temperature control block 7 from the lower surface of the cover portion 3 is required. In addition, the air layer 10 is not limited to a single layer, and may have a structure in which a plurality of layers are stacked in an upper-lower direction. Further, in order to improve airtightness of the air layer 10, an elastic member such as packing may be provided on an inner peripheral surface of the opening portion 3a of the cover portion 3, and a gap with the sample container 4 may be reduced.

### Embodiment 2

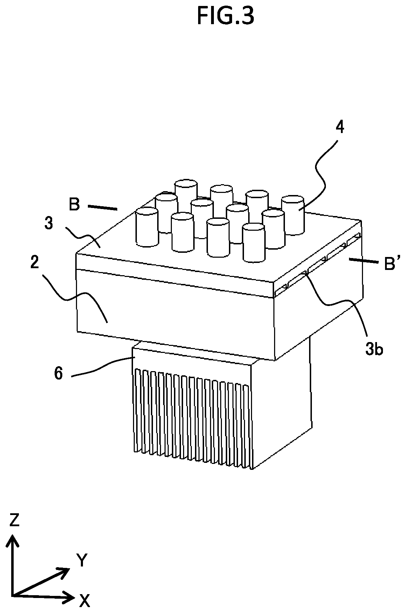

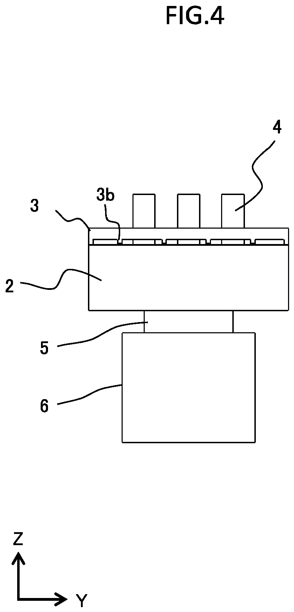

FIG. 3 is a schematic perspective view showing the temperature control device 1 according to Embodiment 2. FIG. 4 is a schematic side view showing the temperature control device 1 according to Embodiment 2. As shown in FIGS. 3 and 4, in the temperature control device 1 according to the present embodiment, a plurality of convex portions 3b that extend in a left-right direction (x direction) are provided on the lower surface of the cover portion 3 in a front-rear direction (y direction). The convex portion 3b may extend in the front-rear direction as long as the convex portion 3b is provided between the adjacent opening portions 3a. In addition, the convex portion 3b may protrude as a result by making a periphery concave.

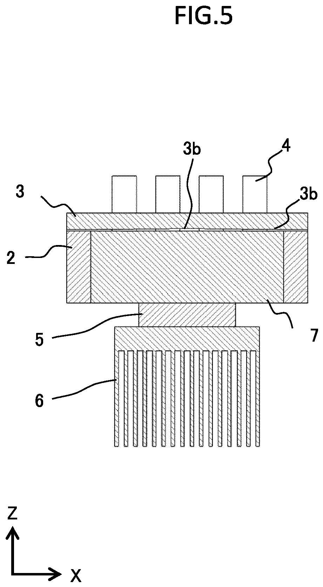

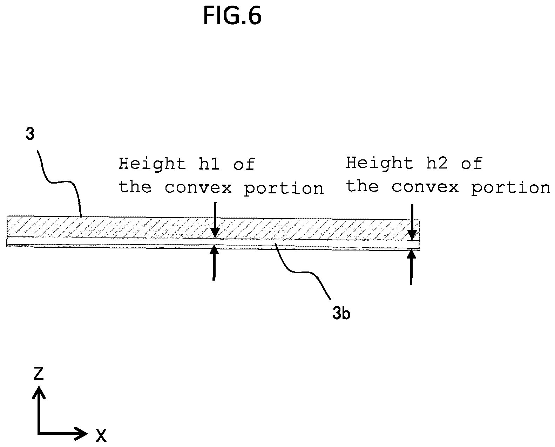

FIG. 5 is a cross-sectional view taken along a line B-B' in FIG. 3. FIG. 6 is an enlarged cross-sectional view showing the convex portion 3b in FIG. 5 in an enlarged manner. As shown in FIGS. 5 and 6, a height h2 of the convex portion 3b near an edge in the left-right direction is larger than a height h1 of the convex portion 3b near a center in the left-right direction. That is, the convex portion 3b that protrudes downward from the cover portion 3 is lower from a center side toward an edge side. The convex portion 3b having such a shape is provided on the lower surface of the cover portion 3, whereby dew condensation water generated due to water vapor contained in the air layer 10 is led to an edge where a gap with the temperature control block 7 is small by a capillary phenomenon. Here, when a hydrophilic treatment is performed on the convex portions 3b of the cover portion 3, discharging the dew condensation water by the capillary phenomenon may be further promoted.

The dew condensation water led to the edge by the convex portion 3b may be guided to a separately provided storage portion or discharging portion, but even if the dew condensation water only accumulates on the edge, the dew condensation water is located at a place away from the opening portion 3a. Therefore, it is possible to prevent splashing of the dew condensation water when inserting and removing the sample container 4. That is, even if the dew condensation water is generated on the upper end surface of the temperature control block 7, contamination of a sample due to the splashing of the dew condensation water can be prevented, and a decrease in testing accuracy can be prevented. The convex portions may be provided on the upper end surface of the temperature control block 7 to discharge the dew condensation water, but since the dew condensation water may accumulate between the convex portions, it is desirable that the convex portions are provided on the lower surface of the cover portion 3.

### Embodiment 3

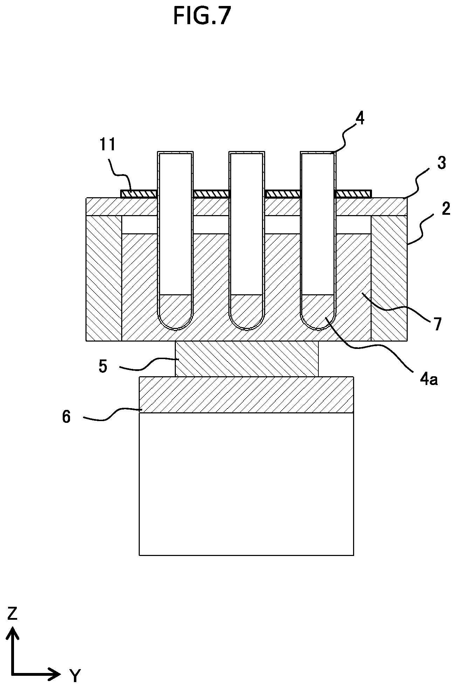

FIG. 7 is a schematic cross-sectional view showing the temperature control device 1 according to Embodiment 3, and corresponds to FIG. 2 in Embodiment 1. As shown in FIG. 7, the temperature control device 1 according to the present embodiment includes a heating part 11 that heats the cover portion 3. Examples of the heating part 11 include a ceramic heater, a film heater, and a Peltier element. In addition, a temperature sensor is provided in the cover portion 3 (not shown). Therefore, the heating part 11 can prevent occurrence of the dew condensation on the upper surface of the cover portion 3 by performing control such that the upper surface of the cover portion 3 is not at the dew point temperature or lower based on a measured temperature of the temperature sensor.

Here, when the temperature control device 1 includes a sensor that measures a temperature and a humidity of the outside air, a dew point temperature of the outside air may be specifically calculated, and the heating part 11 may be controlled to exceed the calculated dew point temperature. When the dew point temperature of the outside air cannot be specifically calculated, a dew point temperature under conditions where the dew condensation is likely to occur can be assumed, and the heating part 11 can also be controlled to exceed the dew point temperature.

Since the heating part 11 according to the present embodiment is provided on the upper surface of the cover portion 3 far from the temperature control block 7, deterioration of performance of cooling the sample container 4 is also prevented. However, the place where the heating part 11 is provided is not limited to the upper surface of the cover portion 3, and the heating part 11 may be provided on the lower surface of the cover portion 3 or outside of the heat insulation material 2.

The heating part 11 according to the present embodiment does not need to be provided on the entire upper surface of the cover portion 3. For example, when at least the upper surface of the cover portion 3 is formed of a material having high thermal conductivity (for example, a metal material such as copper, aluminum, or stainless steel, or a graphite sheet), the entire upper surface of the cover portion 3 can be uniformly heated only by bringing the heating part 11 into contact with only a part of the cover portion 3.

### Embodiment 4

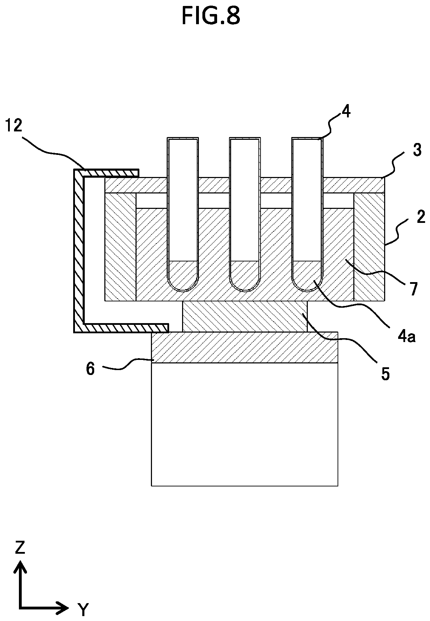

FIG. 8 is a schematic cross-sectional view showing the temperature control device 1 according to Embodiment 4, and corresponds to FIG. 2 in Embodiment 1 and FIG. 7 in Embodiment 3. As shown in FIG. 8, the temperature control device 1 according to the present embodiment includes a heat transfer connection portion 12 that transfers heat from the heat dissipation part 6 to the upper surface of the cover portion 3. One end of the heat transfer connection portion 12 is in contact with the heat dissipation part 6, and the other end of the heat transfer connection portion 12 is in contact with the cover portion 3. In this way, the heat dissipation part 6 and the cover portion 3 are thermally connected, and heat generated by the temperature control part 5 is used to heat the cover portion 3, whereby it is possible to prevent the dew condensation on the upper surface of the cover portion 3.

The heat transfer connection portion 12 is preferably formed of a material having high thermal conductivity, and desirably formed of aluminum, copper, graphite, or the like. Here, when the heat transfer connection portion 12 has a shape with a large cross-sectional area, such as a plate shape, a heat transfer area is increased, and heating the cover portion 3 can also be further promoted. In addition, a heat pipe may be adopted as the heat transfer connection portion 12. Further, the heat transfer connection portion 12 may be connected not only to the heat dissipation part 6, but also to another heat source, for example, a power supply that drives the temperature control device 1, or a semiconductor element provided on a control board of a genetic testing device, and heat of the heat source may be conducted to the cover portion 3.

The examples described above are described in detail in order to describe the invention in an easy-to-understand manner, and are not necessarily limited to including all the described configurations. Further, a part of a configuration of a certain example can also be replaced with a configuration of another example, and a configuration of another example can also be added to a configuration of a certain example. In addition, addition, deletion, and replacement of another configuration can also be made to a part of a configuration of each example.

### Reference Signs List

1: temperature control device
2: heat insulation material
3: cover portion
3a: opening portion
3b: convex portion
4: sample container
4a: solution
5: temperature control part
6: heat dissipation part
7: temperature control block
7a: concave portion
10: air layer
11: heating part
12: heat transfer connection portion

## Claims

1. A temperature control device comprising:
a thermally conductive portion;
a cooling part configured to cool the thermally conductive portion; and
a cover portion having an opening through which a container is inserted and removed while covering an upper side of the thermally conductive portion, wherein
an air layer is formed between the thermally conductive portion and the cover portion.

2. The temperature control device according to claim 1, wherein
a thickness of the air layer is 10 mm or less.

3. The temperature control device according to claim 1, wherein
a convex portion lower from a center side toward an edge side is provided on a lower surface of the cover portion.

4. The temperature control device according to claim 1, further comprising:
a heating part configured to heat the cover portion.

5. The temperature control device according to claim 1, further comprising:
a heat dissipation part configured to dissipate heat generated by the cooling part; and
a heat transfer connection portion configured to transfer heat from the heat dissipation part to the cover portion.
